# EUROPEAN PATENT APPLICATION

(11) **EP 1 900 358 A1**
(43) Date of publication of application: **19.03.2008**
(21) Application number: 06019429.7
(22) Date of filing: 16.09.2006
(51) Int. Cl.: A61K 9/16, A61K 9/20, A61K 31/60

(54) **Pharmaceutical formulations comprising clopidogrel**

(71) Applicant: Cimex Pharma AG, 4253 Liesberg (BL) (CH)
(72) Inventor: Rey, Hélène, 68128 Rosenau (FR); Fischer, Marc, 4106 Therwil (CH); Scheer, Mathias, 79664 Wehr (DE)
(74) Representative: Braun, André jr.

(57) **Abstract**

The formation of melt granulates comprising the drug clopidogrel of the following formula 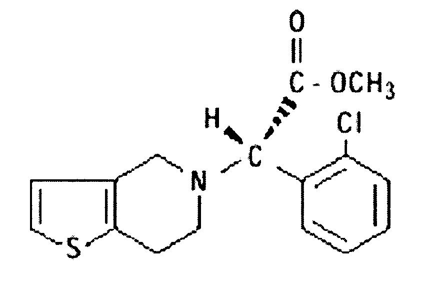 or a pharmaceutically acceptable salt thereof markedly reduces degradation of clopidogrel and its salts on storage.

## Description

### FIELD OF THE INVENTION

The invention relates to certain pharmaceutical formulations comprising clopidogrel or a pharmaceutically acceptable salt thereof, processes for their manufacture and their use in a method of treating warm-blooded animals, including preferably mammals and especially humans.

### BACKGROUND OF THE INVENTION

Clopidogrel is methyl (+)-(S)-alpha-(2-chlorophenyl)-4,5,6,7-tetrahydrothieno[3,2-c]pyridine-5-acetate (= d-enantiomer) of the following formula

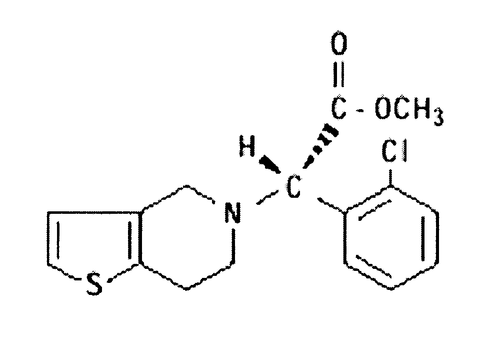

It is marketed in the form of its bisulfate by Sanofi under the trade name PLAVIX® (INN: clopidogrel bisulfate), inter alia as an antiplatelet drug. The world-wide annual sales of PLAVIX in 2005 exceeded 5 billion US dollars.

The racemic form of clopidogrel of the formula 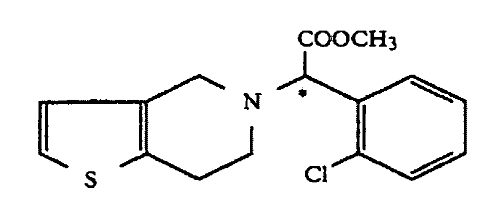 in which the C* is an asymmetric carbon atom in both (R)- and (S)-configuration, and its pharmaceutically acceptable salts are described in Sanofi's patent rights claiming French priority of July 13, 1982 no. FR19820012599. Family members include inter alia US patent 4,529,596 and European patent 99802.

The dextro-rotatory enantiomer (= d-enantiomer) of methyl alpha-5-(4,5,6,7-tetrahydro(3,2-c)thieno-pyridyl)-(2-chlorophenyl)-acetate and certain of its pharmaceutically acceptable salts, like especially its bisulfate, is described in Sanofi's patent rights claiming French priorities of Feb. 17, 1987 (no. FR19870002025) and November 27, 1987 (no: FR19870016516). Family members include inter alia US patent 4,847,265 and European patent 281459.

According to said US patent 4,847,265 (cf. column 1, lines 51-62) many salts of clopidogrel have the drawback of being hygroscopic and difficult to handle on an industrial scale, and their purification proved to be difficult.

### DESCRIPTION OF THE INVENTION

It has now surprisingly been found how the above-mentioned drawback can be overcome. According to the present invention clopidogrel and its pharmaceutically acceptable acid addition salts can be stabilized in pharmaceutical formulations comprising melt granulates.

As shown in the Detailed Description of Experiments hereinafter, the stabilizing effect of the melt granulate formulations is so pronounced that it allows using also those pharmaceutically acceptable acid addition salts of clopidogrel which have been described as being unsuitable in the above-mentioned US patent 4,847,265, e.g. the salt with benzenesulfonic acid, i.e. the besylate. Even more surprisingly, the stabilizing effect of the melt granulate formulations is especially pronounced for clopidogrel salt batches of bad quality. This is especially valuable for salts the purification of which proves to be difficult. For example, as evident from table 2 in Example 10 clopidogrel hydrochloride contains after storage for 30 days at 40 °C and 75% relative humidity nearly twice as much impurities than the clopidogrel hydrochloride stabilized in the melt granulates of the present invention. Hence, the formation of the melt granulates of the present invention surprisingly markedly reduces degradation of clopidogrel and its salts on storage.

The acid addition salts of clopidogrel which can be stabilized according to the present invention are those with pharmaceutically acceptable mineral or organic acids classically used in pharmacy. Appropriate acids are, for example, inorganic acids, such as hydrohalic acid, e. g. hydrochloric, hydrobromic or the like, or sulfuric acid, nitric acid, or phosphoric acid; or suitable organic acids, for example suitable aliphatic acids, like aliphatic mono or dicarboxylic acids, hydroxyalkanoic or hydroxyalkanedioic acids, e.g. acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, or 2-hydroxy-1,2,3-propanetricarboxylic acid; phenylsubstituted alkanoic acids; or suitable aromatic acids, like 2-hydroxybenzoic, or 4-amino-2-hydroxybenzoic acid; or suitable sulfonic acids, like alkanesulfonic acids, e.g. methanesulfonic, or ethanesulfonic acid, or aromatic sulfonic acids, e.g. benzenesulfonic, or 4-methylbenzenesulfonic acid; or cyclohexanesulfamic acid. Preferred acids are e.g. hydrobromic acid, sulphuric acid, phosphoric acid, acetic, benzoic, fumaric, maleic, citric, tartaric, gentisic, dobesilic, methanesulfonic, ethanesulfonic, laurylsulfonic, and paratoluenesulfonic acids, especially hydrochloric acid, and most especially benzenesulfonic acid.

The term "pharmaceutically acceptable salt" as used herein refers to salts which are known to be non-toxic and are commonly used in the pharmaceutical literature. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, beta hydroxybutyrate, chloride, cinnamate, citrate, formate, fumarate, glycolate, heptanoate, lactate, maleate, hydroxymaleate, malonate, nitrate, oxalate, phthalate, phosphate, monohydrogenphosphate, dihydrogenphosphate, metaphosphate, pyrophosphate, propionate, phenylpropionate, salicylate, succinate, sulfate, bisulfate (hydrogensulfate), pyrosulfate, sulfite, bisulfite, sulfonate, p-bromophenylsulfonate, chlorobenzenesulfonate, ethanesulfonate, 2-hydroxyethanesulfonate, methanesulfonate (mesylate), naphthalene-1- sulfonate, naphthalene-2-sulfonate, p-toluenesulfonate (tosylate), xylenesulfonate, tartarate, and the like. Preferred salts are the hydrochloride (chloride) and the benzenesulfonate (besylate).

These salts are prepared in a standard manner, for example by the action of the corresponding acid on clopidogrel base in solution in a solvent from which they precipitate spontaneously or after addition of a non-solvent of the salt.

The term "melt granulate" as used herein defines a solid state dispersion of the active ingredient, i.e. clopidogrel or its pharmaceutically acceptable acid addition salts, i.e. the "drug", in an inert or pharmaceutically acceptable carrier or matrix prepared by a melting (fusion) method. The dispersion of the active ingredient in a solid carrier or diluent by traditional mechanical mixing is not included within the definition of this term. Besides the active ingredient and the carrier or matrix the melt granulates of the present invention can comprise one or more suitable pharmaceutically acceptable excipients in addition.

As used herein, the term "pharmaceutically acceptable carrier" refers preferably to suitable polyethylene glycols (PEGs) as defined hereinbelow. Further carriers which can be used in accordance with the present invention include e.g. Compritol 888 ATO.

Compritol [trade mark] is designated in The National Formulary (NF) as Glyceryl Behenate, and according to the International Nomenclature of Cosmetic Ingredients [INCI] asTribehenin. It is obtainable for example from the company Gattefossé. Compritol is a mixture of esters of glycerol with C₁₆-C₂₂ fatty acids, mainly with C₂₂ fatty acid, i.e. behenic acid.

Polyethylene glycols, also called macrogols, are manufactured by polymerization of ethylene oxide with either water, mono ethylene glycol or diethylene glycol under alkaline catalysis. After the desired molecular weight is reached (according to viscosity measurements) the reaction is terminated by neutralizing the catalyst with an acid. The result are molecules of the structure HO-[CH₂-CH₂-O]ₙ-H wherein n is the number of ethylene oxide units.

As an abbreviation for polyethylene glycols the term "PEG" is used in combination with a numerical value which, within the pharmaceutical industry, indicates the mean molecular weight. Unfortunately, the various pharmacopeias use different nomenclature for some PEG molecular weights. The molecular weights cited herein are in accordance with the European (2002) and US pharmacopeias. The hardness of PEGs increases with increasing molecular weight, however the melting range goes up to a maximum value of about 60 °C only. The most important property of all PEGs is their solubility in water. The lower PEGs up to a molecular weight below about 2000 are hygroscopic. PEGs can be obtained for example from the company Clariant, Basle, Switzerland.

Suitable PEGs for the manufacture of melt granulates according to the present invention have a molecular weight from about 1500 to 35000. Preferred are PEGs having a molecular weight from about 2000 to 20000, especially from about 4000 to 20000, more especially from about 4000 to 10000, e.g. 6000.

The pharmaceutically acceptable excipients which can be present in the melt granulates in addition to the above carrier (like PEG or Compritol 888 ATO) are especially those routinely used in pharmaceutical melt granulates.

Usually, the general method for preparation of a solid dispersion proceeds by a fusion process wherein a pharmaceutically acceptable carrier is mixed with the drug to form an intimate mixture. The mixture is heated at or near the temperature of the melting point or melting range of the pharmaceutically acceptable carrier, thus forming a melt. The molten mixture is then cooled rapidly (shock cooling) to provide a congealed mass which may subsequently be milled to produce a powder.

Alternatively, the pharmaceutically acceptable carrier can be heated to molten condition upon which the drug can be added to the molten carrier or vice versa under stirring or mixing, e.g. in a high shear mixer, like a Diosna, thus forming a molten homogeneous melt. Preferably, the molten carrier is added to the drug under stirring or mixing.

Lastly, the process proceeds by cooling the molten homogeneous melt to form a solid state dispersion. Cooling can be effected by conventional methods or, according to the preferred method of the present invention, by shock cooling, for example by adding, in a high shear mixer, dry ice, i.e. solid carbon dioxide having a temperature of about -78 °C, preferably in the form of a kind of snow ("dry snow"), to the dispersion which thereupon breaks apart into many small particles which are sieved, e.g. through 1-2 mm.

The ratio of the drug to the pharmaceutically acceptable carrier can be varied over a wide range and depends on the concentration of the drug required in the pharmaceutical dosage form ultimately administered. However, the preferred range of the drug in the solid dispersion is about 10% to 90% of the total solid dispersion weight, more preferable is about 20% to 90%, even more preferable is about 40% to 90%, especially about 50% to 90%, e.g. 50, 60, 70 or 80%, of the total dispersion weight.

The present invention also provides a pharmaceutical composition comprising in addition to the melt granulate as defined above (further) pharmaceutically acceptable excipients (one or more of which may, as described above, be also included in the melt granulate). Examples of pharmaceutically acceptable excipients include diluents, binders, disintegrants, coloring agents, flavoring agents, lubricants and/or preservatives. The pharmaceutical composition may be formulated by conventional methods of admixture such as blending, filling, granulation and compressing.

Besides acting as carriers for the active ingredient, PEGs also act as lubricants and binders during the tablet processing and solid PEGs are also frequently used in tablet coatings. These other uses of PEGs are distinguishable and have to be clearly distinguished from the use in the formation of melt granulates.

The medicine of the invention can be made available for oral administration for example in the form of tablets, which may be coated, like e.g. sugar-coated, or capsules.

The melt granulates and pharmaceutical compositions of the present invention may be used to treat warm-blooded animals, including mammals and humans, for the same indications which can be treated by PLAVIX, e.g. on account of its interesting inhibitory properties towards platelet aggregation and its interference in the mechanism of formation of arterial and venous thromboses. The medicine of the invention can be usefully administered in the treatment and prevention of platelet disorders, including for example also those due to extracoporeal blood circuits or the consequence of complications in atheroma. Mainly, the melt granulates of the present invention may be used for the manufacture of pharmaceutical compositions for reduction of atherothrombotic events in case of recent myocardial infarction, recent stroke, established peripheral arterial disease, and acute coronary syndrome.

The composition is usually presented as a unit dose composition containing from 30 to 100 mg, more usually from 60 to 90 mg, preferably 75 mg of clopidogrel. Such composition is normally administered once daily to a warm-blooded animal, including a human, of about 70 kg body weight, or as recommended for PLAVIX. As usual, the exact dose to be administered depends inter alia on the age, health and weight of the recipient; the nature and extent of the symptoms; the kind of concurrent treatment; the frequency of treatment; and the effect desired. The treatment of patients with acute coronary syndrome should be initiated with a single 300 mg loading dose of clopidogrel and then continued at 75 mg once daily. In addition, Aspirin and/or heparin may be administered.

The following examples illustrate the invention.

### Abbreviations:

rpm: rounds per minute
s: second(s)

### DETAILED DESCRIPTION OF EXPERIMENTS

### Example 1: melt granulate consisting of 10 % by weight of PEG 6000 and 80% of clopidogrel besylate

Melt 22.4 g of PEG 6000 using a hot plate, until it has reached 80°C. Place 201.3 g of clopidogrel besylate in a high-shear mixer. While stirring (400 rpm), pour the melted PEG into the bowl containing clopidogrel. Add 50 g of dry ice (in the form of dry snow) and increase the speed to 3000 rpm for 30 seconds. The resulting granulate has a temperature of about 15°C. and is sieved through 2.0 and 1.0 mm.

Clopidogrel besylate used as a starting material is prepared as follows:

### Step 1.1: Manufacture of clopidogrel besylate

7 g (0.0228 mole) of dextro-rotatory methyl alpha-5(4,5,6,7-tetrahydro(3.2-c)thieno pyridyl) (2-chlorophenyl)acetate (i.e. clopidogrel in the form of its free base) is dissolved in 100 ml of diethyl ether. To the obtained solution 30 ml of diethyl ether containing 4.78 g (0.03 mole) of anhydrous benzene sulfonic acid are added at room temperature (i.e. at 28°C - 31°C). After complete addition the reaction mass is maintained at room temperature (28°C - 31°C) for 14 hours. The white solid is collected by filtration and dried at 50-60 °C in a vacuum oven whereupon 2.2 g of clopidogrel besylate are obtained in crystalline form (99.82% pure according to HPLC); DSC: onset at 127.45°C, peak at 135.69°C.

### Example 2: melt granulate consisting of 30 % PEG 6000 and 70% clopidogrel besylate

Analogously as described in Example 1 the above melt granulate is obtained from 86.4 g of clopidogrel besylate, 201.3 g of PEG 6000, and 80 g of dry snow.

### Example 3: melt granulate consisting of 50 % PEG 6000 and 50% clopidogrel besylate

Analogously as described in Example 1 the above melt granulate is obtained from 201.3 g of clopidogrel besylate and 201.3 g of PEG 6000, adding between 130 and 150 g of dry snow and increasing the speed of the mixer to 3000 rpm for 1 minute.

### Example 4: melt granulate consisting of 50 % PEG 4000 and 50% clopidogrel besylate

Melt 201.3 g of PEG 4000 using a hot plate, until it has reached 76°C. Place 201.3 g of clopidogrel besylate in a high-shear mixer. While stirring (600 rpm), pour the melted PEG in the bowl containing the clopidogrel. Add 150 g of dry ice and increase the speed to 1800 rpm for 1 mn. The resulting granulate has a temperature of 17.6°C, and is sieved through 2.00 and 1.0 mm

### Example 5: melt granulate consisting of 50 % PEG 20000 and 50% clopidogrel besylate

201.3 g of PEG 20000 are melted using a hot plate, until it has reached 100°C. 201.3 g of clopidogrel besylate are placed into a high-shear mixer. While stirring (400 rpm) the melted PEG is poured into the bowl containing the clopidogrel. 50 g of dry ice are added and the speed of the mixer is increased to 3000 rpm for 1 minute. The resulting granulate has a temperature of 15°C, and is sieved through 2.0 and 1.0 mm.

### Example 6: melt granulate consisting of 50 % by weight of PEG 35000 and 50% clopidogrel besylate

Melt 201.3 g of PEG 35000 using a hot plate, until it has reached 100°C. Place 201.3 g of clopidogrel besylate in a high-shear mixer. While stirring (400 rpm), pour the melted PEG into the bowl containing the clopidogrel. Add 100 g of dry ice and increase the speed to 3000 rpm for 2 minutes. The resulting granulate has a temperature of 30°C, and is sieved through 2.0 and 1.0 mm.

### Example 7: melt granulate consisting of 50 % by weight of PEG 6000 and 50% clopidogrel hydrochloride

Melt 10.0 g of PEG 6000 using a 78°C water bath until it has reached 74°C. Place 10.0 g of clopidogrel hydrochloride into a mortar. While manually stirring, pour the melted PEG in the mortar containing the clopidogrel hydrochloride. Stir another 2 minutes, then let the resulting mass cool down to room temperature, before grinding it.

### Example 8: melt granulate consisting of 50 % by weight of Compritol 888 ATO and 50% clopidogrel hydrochloride

Melt 10.0 g of Compritol 888 ATO using a 78°C water bath until it has reached 74°C. Place 10.0 g of clopidogrel hydrochloride into a mortar. While manually stirring, pour the melted Compritol into the mortar containing the clopidogrel. Stirr another 2 minutes, then let the resulting mass cool down to room temperature, before grinding it.

### Example 9: Storage stability of clopidogrel besylate and melt granulates containing it

The stability on storage of clopidogrel besylate salt (CLO˙Bes) is examined in comparison to melt granulates containing it. The main degradation product is alpha-(2-chlorophenyl)-4,5,6,7-tetrahydrothieno[3,2-c]pyridine-5-acetic acid, hereinafter designated as "ImpA'' in accordance with USP 29. In addition, the **sum** of all **imp**urities ("SumImp") is determined. Both ImpA and SumImp are expressed as percent by weight of the amount of the clopidogrel salt at the beginning of the experiment on day 0 and are determined using the HPLC-system described below.

In the below table 1 for example the term "10% PEG 6000" means the melt granulate consisting of 10% by weight PEG 6000 and 90% by weight clopidogrel besylate, 30% PEG 6000 means the melt granulate consisting of 30% by weight PEG 6000 and 70% by weight clopidogrel besylate, etc.

**Table 1**

| **Tested material** | **Day 0** | | **Day 15** | |
|---|---|---|---|---|
| | | | at 50 °C and 75% relative humidity | |
| | **ImpA [%]** | **SumImp [%]** | **ImpA [%]** | **SumImp [%]** |
| CLO˙Bes | 0.09 | 0.24 | 0.34 | 0.52 |
| 10% PEG 6000 | 0.08 | 0.23 | 0.21 | 0.39 |
| 30% PEG 6000 | 0.09 | 0.24 | 0.19 | 0.36 |
| 50% PEG 6000 | 0.08 | 0.24 | 0.18 | 0.38 |
| 50% PEG 20000 | 0.09 | 0.25 | 0.19 | 0.38 |
| 50% PEG 35000 | 0.09 | 0.24 | 0.19 | 0.36 |

As evident from the above table 1 clopidogrel besylate (CLO Bes) contains after storage for 15 days at 50 °C and 75% relative humidity on an average about 79 % (0.34/0.19 = 1.79) more of the impurity ImpA and about 41 % (0.52/0.37 = 1.41) more total impurities SumImp than the clopidogel besylate stabilized in the above melt granulates. The values of 0.19 and 0.37 mentioned herein above in parentheses and used for calculation are the averages of the values listed for the granulates in the columns ImpA and SumImp, respectively, on day 15.

### The above-mentioned HPLC system is as follows:

Instruments and operating conditions:
Column: 250 mm × 4.6 mm Modulo-Cart QS Uptisphere HDO, 3 µm
Detector: UV Detector or DAD Detector
Eluent A: 0.01 M sodium phosphate dibasic aqueous solution (1.78 g Na₂HPO₄ * 2 H₂O/l) adjusted to pH 3.0 ± 0.05 with phosphoric acid 85%
Eluent B: Acetonitril
Mobile phase: 35% eluent A + 65% eluent B
Gradient:

| minutes | Eluent A [%] | Eluent B %] |
|---|---|---|
| 0.00 | 75 | 25 |
| 0.10 | 65 | 35 |
| 1.50 | 65 | 35 |
| 11.50 | 35 | 65 |
| 40.00 | 20 | 80 |
| 50.00 | 20 | 80 |
| 51.00 | 75 | 25 |

Operating conditions:
Flow rate: 0.4 ml/min
Column temperature: 20°C
Injection volume: 10 µl
Wavelength: 235 nm
Runtime: 65 min
Retention time:

| Compound | Retention time [minutes] | Relative retention time (vs. clopidogrel) |
|---|---|---|
| Benzenesulfonic acid | 7.6 | 0.18 |
| Diluent | 8.6 | 0.21 |
| Gradient | 11.0 | 0.27 |
| Impurity A | 12.3 | 0.30 |
| Clopidogrel | 41.3 | 1.00 |
| Gradient | 48.0 | 1.16 |

### Preparation of reference and sample solutions:

### Reference solution

Approximately 55.92 mg of clopidogrel besilate reference substance are weighed into a 50 ml volumetric flask. Add 20 ml of mobile phase and sonicate in an ultrasonic bath at ambient temperature until the substance is dissolved (approx. 5 min). Make up to the mark with mobile phase. The concentration of clopidogrel besilate is 0.75 mg/ml as free base. Two independent reference solutions are prepared.

### Sample solution for 75 mg tablets

Weigh 10 tablets and transfer them quantitatively into a 250 ml volumetric flask. Dissolve and make up to the mark with mobile phase. Dilute 25.0 ml to 100.0 ml with mobile phase. Filter through a 0.45 µm PTFE-filter in a HPLC vial. The concentration of clopidogrel is 0.75 mg/ml as free base. Two independent sample solutions are prepared.

### Example 10: Storage stability of clopidogrel hydrochloride

The stability on storage of clopidogrel hydrochloride salt (CLO˙HCl) has been examined in comparison to melt granulates containing it. The main degradation product is alpha-(2-chlorophenyl)-4,5,6,7-tetrahydrothieno[3,2-c]pyridine-5-acetic acid, hereinafter designated as "ImpA" (**imp**urity **a**cid). In addition, the **sum** of all **imp**urities ("SumImp") is determined by HPLC analogously as described above for clopidogrel besylate. Both ImpA and SumImp are expressed as percent by weight of the amount of the clopidogrel salt at the beginning of the experiment on day 0. The granulates mentioned in the below table 2 are melt granulates consisting of
50 % by weight of PEG 6000 and 50% clopidogrel hydrochloride, and
50 % Compritol 888 ATO and 50% clopidogrel hydrochloride, respectively.

**Table 2**

| **Tested material** | **Day 0** | | **Day 30** | |
|---|---|---|---|---|
| | | | at 40 °C and 75% relative humidity | |
| | **ImpA [%]** | **SumImp [%]** | **ImpA [%]** | **SumImp [%]** |
| CLO˙HCl | 0.18 | 0.58 | 1.28 | 1.99 |
| 50% PEG 6000 | 0.13 | 0.49 | 0.65 | 1.03 |
| 50 % Compritol | 0.15 | 0.57 | 0.66 | 1.04 |

As evident from the above table 2 clopidogrel hydrochloride (CLO HCL) contains after storage for 30 days at 40 °C and 75% relative humidity on an average about 95 % (1.28/0.655 = 1.95) more of the impurity ImpA (i.e. nearly twice as much) and about 92 % (1.99/1.035 = 1.92) more total impurities SumImp than the clopidogrel hydrochloride stabilized in the above melt granulates.

### Example 11: manufacture of tablets comprising a melt granulate containing 50% by weight of clopidogrel besylate and 50% by weight of PEG 6000

In a first step, the granulate is mixed with the excipients in order to form the final blend. 44.74 g of granulate are placed in a container. 26.74 g of Avicel® PH200 (microcrystalline cellulose), 3.80 g of Plasdone® XL (1-ethenylpyrrolidin-2-one) and 0.72 g of Cutina® HR (Hydrogenated Castor Oil) are added. The container is sealed and then the blend is mixed for 10 minutes so that it becomes homogeneous.

In a second step, the final blend is tabletted using 10 mm R9 round, biconvex punches. Each unit has a mass of 380 mg, and is tabletted so that its hardness is between 80 and 100N and its thickness between 5.4 and 6.0 mm.

## Claims

1. Melt granulate comprising clopidogrel or a pharmaceutically acceptable salt thereof.

2. Melt granulate according to claim 1 comprising a pharmaceutically acceptable salt of clopidogrel.

3. Melt granulate according to claim 1 comprising clopidogrel besylate or clopidogrel hydrochloride.

4. Melt granulate according to any one of claims 1-3 comprising a polyethylene glycol having a mean molecular weight from 1500 to 35000.

5. Melt granulate according to any one of claims 1-3 comprising a polyethylene glycol having a mean molecular weight from 4000 to 10000.

6. Melt granulate according to any one of claims 1-4 containing from 10 to 90 per cent by weight of clopidogrel or a pharmaceutically acceptable salt thereof.

7. Melt granulate according to any one of claims 1-5 containing from 10 to 90 per cent by weight of a pharmaceutically acceptable salt of clopidogrel and (100 minus x) per cent by weight of a polyethylene glycol having a mean molecular weight of from 2000 to 30000, wherein x is the content of the pharmaceutically acceptable salt of clopidogrel expressed as per cent by weight.

8. Process of manufacture of a melt granulate as claimed in any one of claims 1-6 wherein dry ice is added to the hot melt granulate for shock cooling.

9. Pharmaceutical composition comprising a melt granulate as claimed in any one of claims 1-7 together with pharmaceutical excipients.

10. Method of reducing atherothrombotic events in a human patient in case of recent myocardial infarction, recent stroke, established peripheral arterial disease, and acute coronary syndrome by administering to said patient an atherothrombotic events reducing effective amount of a pharmaceutical composition as claimed in claim 9.
